# EUROPÄISCHE PATENTANMELDUNG

(11) **EP 2 191 818 A1**
(43) Veröffentlichungstag der Anmeldung: **02.06.2010**
(21) Anmeldenummer: 08020775.6
(22) Anmeldetag: 28.11.2008
(51) Int. Cl.: A61K 8/92, A61Q 15/00

(54) **Antiperspirant/Desodorant-Zubereitungen mit verbesserten sensorischen Eigenschaften**

(71) Anmelder: Cognis IP Management GmbH, 40589 Düsseldorf (DE)
(72) Erfinder: Brüning, Stefan, 40593 Düsseldorf (DE); Kawa, Rolf, 40789 Monheim (DE); Weichold, Catherine, 52062 Aachen (DE)
(74) Vertreter: Gittinger, Andreas

(57) **Zusammenfassung**

Die vorliegende Anmeldung betrifft Antiperspirant/Desodorant-Zubereitungen mit hohem Anteil an festen Wachsen in der lipophilen Phase, die hinsichtlich ihrer Sensorik vesbessert sind, sowie ein Verfahren zur Verbesserung der Sensorik von Antiperspirant/Desodorant-Zubereitungen.

## Beschreibung

### Gebiet der Erfindung

Die vorliegende Erfindung betrifft Antiperspirant/Desodorant-Zubereitungen mit hohem Anteil an festen Wachsen in der lipophilen Phase, die hinsichtlich ihrer Sensorik verbessert sind, sowie ein Verfahren zur Verbesserung der Sensorik von Antiperspirant/Desodorant-Zubereitungen.

### Stand der Technik

Antiperspirant/Desodorant-Formulierungen des Marktes haben in der Regel hinsichtlich der Schutzwirkung gegen Schweißgeruch eine ausreichende Leistung. Zubereitungen gemäß Stand der Technik weisen aber nach der Anwendung ein lang andauemdes feuchtes Gefühl unter der Achsel auf, das zudem mit einem klebrigen Endgefühl verbunden ist. Dies gilt insbesondere für O/W Roll-on Formulierungen.

Es gibt zwar Lösungsansätze in der Literatur, um ein trockenes Endgefühl zu erreichen. Diese beziehen sich jedoch ausschließlich auf wasserfreie Systeme (W. Umbach, Kosmetik und Hygiene, Wiley-VCH, 3. Auflage, S. 371), die zwar richtigerweise ein trockenes Hautgefühl nach dem Auftragen aufweisen, was aber nach kurzer Zeitz stumpf und unangenehm erscheint.

Aufgabe der vorliegenden Erfindung war es, Antiperspirant/Desodorant-Formulierungen zur Verfügung zu stellen, die möglichst direkt nach Anwendung in die Haut einziehen und die ein trockenes, nicht klebriges und glattes Endgefühl hinterlassen, wobei die Schutzwirkung als Antitperspirant/Desodorant beibehalten wird.

### Beschreibung der Erfindung

Gegenstand der vorliegenden Erfindung sind Antiperspirant/Desodorant-Zubereitungen mit folgenden Bestandteilen:
a. einem Antiperspirant-/Desodorant-Wirkstoff, und
b. einer lipophilen Phase, die zu 50 Gew.-% oder mehr, bevorzugt etwa 70 bis 80 Gew.-%, bezogen auf das Gesamtgewicht der lipophilen Phase, aus Wachsen mit einem Schmelzpunkt von > 30°C gebildet ist.

Die vorliegende Erfindung betrifft auch ein Verfahren zur Verbesserung der sensorischen Eigenschaften einer Antiperspirant/Desodorant-Zubereitung, insbesondere ein Verfahren zur Verbesserung des Nässe- und/oder Klebrigkeitsgefühls direkt nach dem Auftragen, **dadurch gekennzeichnet, dass** man die lipophile Phase derart einstellt, dass diese etwa 50 Gew.-% oder mehr, bevorzugt etwa 70 - 80 Gew.-%, bezogen auf das Gewicht der lipophilen Phase, Wachs(e) mit einem Schmelzpunkt von > 30°C enthält.

Überraschenderweise wurde gefunden, dass bei einer Erhöhung der Wachsanteils in der lipophilen Phase auf 50 Gew.-% oder mehr - in Bezug auf die lipophile Phase - die Antiperspirant/Desodorant-Zubereitungen schnell einziehen, also schnell ein deutlich trockeneres und glattes Endgefühl aufweisen und nicht kleben. Dabei wird die Schutzwirkung, d. h. die Wirkung als Antiperspirant/Desodorant, nicht beeinträchtigt. Eine solche Wirkung durch eine Erhöhung des Wachsanteils in der lipophilen Phase zu erzielen, war im Hinblick auf bekannte AP/Deo-Formulierungen nicht zu erwarten.

Die erfindungsgemäßen Zubereitungen liegen üblicherweise als Emulsionen vor, d. h. sie enthalten Emulgatoren und ggf. Coemulgatoren. Es sind erfindungsgemäß aber auch Ausführungsformen erfasst, die ohne Emulgatoren und Coemulgatoren auskommen, wie bspw. Zubereitungen die Polyquatemiumverbindungen wie Polyquatemium 37 enthalten.

### Lipophile Phase

Zur lipophilen Phase gehören erfindungsgemäß die wasserunlöslichen Bestandteile Öle und Wachse. Erfindungsgemäß gehören zur lipophilen Phase nicht Emulgatoren und Coemulgatoren. Nachfolgend werden Möglichkeiten der Bestandteile der lipophilen Phase, also der Wachse und Öle, angegeben. Dabei sollen erfindungsgemäß solche Öle und Wachse, die in der jeweiligen Zubereitung eine Emulgator- und/oder Coemulgatorwirkung aufweisen, nicht mit umfasst sein. Dadurch kann, insbesondere für die Wachskomponenten, gewährleistet werden, dass diese sich innerhalb der lipophilen Phase befinden und nicht an der Grenzfläche zur wässrigen Phase, was erfindungsgemäß bevorzugt ist.

Die lipophile Phase ist erfindungsgemäß zu 50 Gew.-% oder mehr, bevorzugt etwa 60 bis etwa 85 Gew.-%, weiterhin bevorzugt etwa 70 bis etwa 80 Gew.-% - bezogen auf das Gesamtgewicht der lipophilen Phase - aus Wachs(en) mit einem Schmelzpunkt oberhalb 30°C gebildet. Der Restbestandteil der lipophilen Phase wird üblicherweise durch bei Raumtemperatur flüssige Öle gebildet.

Die lipophile Phase ist üblicherweise in einem Anteil von 2 bis 15 Gew.-% oder bevorzugter 3 bis 8 Gew.-%., bezogen auf das Gesamtgewicht der Antiperspirant-/Desodorant-Zubereitung, enthalten.

Unter dem Begriff **Wachs** (synonym verwendet: Wachskomponente) werden üblicherweise und erfindungsgemäß alle natürlichen oder künstlich gewonnenen Stoffe und Stoffgemische mit folgenden Eigenschaften verstanden: sie sind von fester bis brüchig harter Konsistenz, grob bis feinteilig, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30°C oder darüber schmelzen.

Als Wachse können erfindungsgemäß Fette und fettähnliche Substanzen mit wachsartiger Konsistenz eingesetzt werden, solange sie den geforderten Schmelzpunkt haben. Hierzu gehören u.a. Fette (Triglyceride), sowie natürliche und synthetische Wachse oder beliebige Gemische dieser Substanzen.

Als Wachskomponenten können auch C₁₄-C₄₀-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise **natürliche pflanzliche Wachse,** wie Candelillawachs, Camaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und **tierische Wachse,** wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die **Mineralwachse,** wie z. B. Ceresin und Ozokerit oder die petrochemischen Wachse, wie z. B. Petrolatum, Paraffinwachse und Mikrowachse. Als Wachskomponente sind auch **chemisch modifizierte Wachse,** insbesondere die Hartwachse, wie z. B. Montanesterwachse, Sasolwachse und hydrierte Jojobawachse einsetzbar. Zu den **synthetischen Wachsen,** die erfindungsgemäß einsetzbar sind, zählen beispielsweise wachsartige Polyalkylenwachse und Polyethylenglycolwachse. Pflanzliche Wachse sind erfindungsgemäß bevorzugt

Als Wachse eignen sich weiterhin **Perlglanzwachse.** Als Perlglanzwachse, insbesondere für den Einsatz in tensidischen Formulierungen, kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierie Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als Wachskörper mit einem Erstarrungspunkt oberhalb von >30°C kommen beispielsweise in Frage: Alkylenglycolester, Fette, Partialglyceride, Fettalkoholethoxylate, Fettsäurealkanolamide, Ester von mehrwertigen, gegebenenfalls hydroxysubstituierten Carbonsäuren mit Fettalkoholen mit 6 bis 22 Kohlenstoffatomen, Fettstoffe, wie beispielsweise Fettalkohole, Fettketone, Fettaldehyde, Fettether und Fettcarbonate, die in Summe mindestens 12, vorzugsweise mindestens 18 Kohlenstoffatome aufweisen; Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen, sowie deren Mischungen.
Im Folgenden sind verwendbare Substanzklassen und Beispiele hierfür noch ausführlicher beschrieben:

**Alkylenglycolester.** Bei den Alirylenglycolestem handelt es sich üblicherweise um Mono- und/oder Diester von Alkylenglycolen die der Formel (1) entsprechen,

R¹CO(OA)ₙOR² (1)

in der R¹CO für einen linearen oder verzweigten, gesättigten oder ungesättigten Acylrest mit 6 bis 22 Kohlenstoffatomen, R² für Wasserstoff oder R¹CO und A für einen linearen oder verzweigten Alkylenrest mit 2 bis 4 Kohlenstoffatomen und n für Zahlen von 1 bis 5 steht. Typische Beispiele sind Mono- und/oder Diester von Ethylenglycol, Propylenglycol, Diethylenglycol, Dipropylenglycol, Triethylenglycol oderTetraethylenglycol mit Fettsäuren mit 6 bis 22, vorzugsweise 12 bis 18 Kohlenstoffatomen, z.B.: 2-Ethylhexansäure, Caprinsäure, Caprylsäure, Myristinsäure, Palmitinsäure, Palmoleinsäure, Stearinsäure, lso-Laufinsäure, lsotridecansäure, Elaldinsäure, Petroselinsäure, Linolsäure, Linolensäure, Elaeostearinsäure, Stearinsäure, Ölsäure, Arachinsäure, Gadoleinsäure, Behensäure und Erucasäure sowie deren technische Mischungen.

**Fette.** Unter Fetten versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar sind so genannte gehärtete Fette und Öle, die durch Partiathydrierung gewonnen werden. Pflanzliche gehärtete Fette und Öle sind bevorzugt, z. B. gehärtetes Rizinusöl, Erdnussöl, Sojaöl, Rapsöl, Rübsamenöl, Baumwollsaatöl, Sojaöl, Sonnenblumenöl, Palmöl, Palmkemöl, Leinöl, Mandelöl, Maisöl, Olivenöl, Sesamöl, Kakaobutter und Kokosfett.

Geeignet sind u.a. die Dreifachester von Glycerin mit C₁₂-C₆₀-Fettsäuren und insbesondere C₁₂-C₃₆-Fettsäuren. Hierzu zählt gehärtetes Rizinusöl, ein Dreifachester aus Glycerin und einer Hydroxystearinsäure, der beispielsweise unter der Bezeichnung Cutina^{®} HR im Handel ist. Ebenso geeignet sind Glycerintristearat, Glycerintribehenat (z. B. Syncrowax^{®} HRC), Glycerintripalmitat oder die unter der Bezeichnung Syncrowax^{®} HGLC bekannten Triglycerid-Gemische, mit der Vorgabe, dass der Schmelzpunkt der Wachskomponente bzw. des Gemisches bei 30°C oder darüber liegt.

**Partialglyceride.** Partialglyceride, die als Wachskörper in Frage kommen, stellen Mono- und/oder Diester des Glycerins mit Fettsäuren, nämlich beispielsweise Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen dar. Sie folgen der Formel (II): 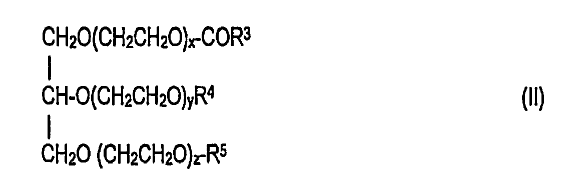 in der R³CO für einen linearen oder verzweigten Acylrest mit 6 bis 22 Kohlenstoffatomen, R⁴ und R⁵ unabhängig voneinander für Wasserstoff oder R³CO, x, y und z in Summe für 0 oder für Zahlen von 1 bis 30 und X für ein Alkali-oder Erdalkalimetall mit der Maßgabe steht, dass mindestens einer der beiden Reste R⁴ und R⁵ Wasserstoff darstellt. Typische Beispiele sind Laurinsäuremonoglycerid, Laurinsäurediglycerid, Kokosfettsäuremonoglycerid, Kokosfettsäuretriglycerid, Palmitinsäuremonoglycerid, Palmitinsäuretriglycerid, Stearinsäuremonoglycerid, Stearinsäurediglycerid, Isostearinsäuremonoglycerid, Isostearinsäurediglycerid, Ölsäuremonoglycerid, Ölsäurediglycerid, Talgfettsäuremonoglycerid, Talgfettsäurediglycerid, Behensäuremonoglycerid, Behensäurediglycerid, Erucasäuremonoglycerid, Erucasäurediglycerid sowie deren technische Gemische, die untergeordnet aus dem Herstellungsprozess noch geringe Mengen an Triglycerid enthalten können.

**Fettsäurealkanolamide**. Fettsäurealkanolamide, die als Wachskörper in Frage kommen, entsprechen der Formel (III),

R³CO-NR⁴-B-OH (III)

in der R³CO für einen linearen oder verzweigten, gesättigten oder ungesättigten Acylrest mit 6 bis 22 Kohlenstoffatomen, R⁴ für Wasserstoff oder einen gegebenenfalls hydroxysubstituierten Alkylrest mit 1 bis 4 Kohlenstoffatomen und B für eine lineare oder verzweigte Alkylengruppe mit 1 bis 4 Kohlenstoffatomen steht. Typische Beispiele sind Kondensationsprodukte von Ethanolamin, Methylethanolamin, Diethanolamin, Propanolamin, Methylpropanolamin und Dipropanolamin sowie deren Mischungen mit Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren Mischungen.

**Mehrwertige Carbonsäure- und Hydroxycarbonsäureester.** Als Wachskörper kommen weiterhin Ester von mehrwertigen, gegebenenfalls hydroxysubstituierten Carbonsäuren mit Fettalkoholen mit 6 bis 22 Kohlenstoffatomen in Frage. Die Wachskomponente kann gewählt werden aus der Gruppe der Wachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispiel solcher Ester sind die C₁₆-C₄₀-Alkylstearate, C₂₀-C₄₀-Alkylstearate (z. B. Kesterwachs K82H), C₂₀-C₄₀Dialkylester von Dimersäuren, C₁₈-C₃₈-Alkylhydroxystearoylstearate oder C₂₀-C₄₀-Alkylerucate. Ferner sind C₃₀-C₅₀-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetylester, Cetearylbehenat und Behenylbehenat einsetzbar. Auch Fettsäurepartialglyceride, d. h. technische Mono- und/oder Diester des Glycerins mit Fettsäuren mit 12 bis 18 Kohlenstoffatomen wie etwa Glycerinmonoldifaurat, -palmitat, - myristat oder-stearat kommen hierfür in Frage. Als Säurekomponente dieser Ester kommen beispielsweise Malonsäure, Maleinsäure, Fumarsäure, Adipinsäure, Sebacinsäure, Azelainsäure, Dodecandisäure, Phthalsäure, Isophthalsäure und insbesondere Bernsteinsäure sowie Äpfelsäure, Citronensäure und insbesondere Weinsäure und deren Mischungen in Betracht. Die Fettalkohole enthalten 6 bis 22, vorzugsweise 12 bis 18 und insbesondere 16 bis 18 Kohlenstoffatome in der Alkylkette. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Die Ester können als Voll-oder Partialester vorliegen, vorzugsweise werden Mono-und vor allem Diester der Carbon- bzw. Hydroxycarbonsäuren eingesetzt. Typische Beispiele sind Bemsteinsäuremono- und -dilaurylester, Bemsteinsäuremono- und -dicetearlyester, Bernsteinsäuremono- und -distearylester, Weinsäuremono- und -dilaurylester, Weinsäuremono- und -dikokosalkylester, Weinsäuremono- und -dicetearylester, Citronensäuremono-, -di- und -trilaurylester, Citronensäuremono-, -di- und -trikokosalkylester sowie Citronensäuremono-, -di- und -tricetearylester.

**Fettalkohole.** Als weitere Gruppe von Wachskörpem können langkettige Fettalkohole eingesetzt werden, die der Formel (IV) entsprechen,

R⁸OH (IV)

in der R⁸ für einen linearen Alkylrest mit 12 bis 50, becorzugt 18 bis 48, bevorzugter 24 bis 36 Kohlenstoffatomen, steht.

Zu den erfindungsgemäß als Wachskomponente einsetzbaren **Fettalkoholen** zählen die C₁₂-C₅₀-Fettalkohole. Die Fettalkohole können aus natürlichen Fetten, Ölen und Wachsen gewonnen werden, wie beispielsweise Myristylalkohol, 1-Pentadecanol, Cetylalkohol, 1-Heptadecanol, Stearylalkohol, Hydroxystearylalkohol,1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Aber auch ungesättigte, verzweigte oder unverzweigte Fettalkohole können erfindungsgemäß als Wachskomponente verwendet werden, solange sie den geforderten Schmelzpunkt aufweisen. Erfindungsgemäß einsetzbar sind auch Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegier-Synthese (Alfole) oder die teilweise verzweigten Alkohole aus der Oxosynthese (Dobanole) verwendet werden. Erfindungsgemäß besonders bevorzugt geeignet sind C₁₄-C₂₂-Fettalkohole, die beispielsweise von der Cognis Deutschland GmbH unter der Bezeichnung Lanette 18 (C₁₈-Alkohol), Lanette 16 (C₁₆-Alkohol), Lanette 14 (C₁₄-Alkohol), Lanette O (C₁₆/C₁₈-Alkohol) und Lanette 22 (C₁₈/C₂₂-Alkohol) vermarktet werden. Fettalkohole verleihen den Zubereitungen ein trockeneres Hautgefühl als Triglyceride und sind daher gegenüber letzteren bevorzugt.

**Fettketone.** Fettketone, die als Wachskörper in Betracht kommen, entsprechen vorzugsweise der Formel (V),

R⁹-CO-R¹⁰ (V)

in der R⁹ und R¹⁰ unabhängig voneinander für Alkyl- und/oder Alkenylreste mit 1 bis 22 Kohlenstoffatomen stehen, mit der Maßgabe, dass sie in Summe mindestens 12 und vorzugsweise 18 bis 48 Kohlenstoffatome aufweisen. Die Ketone können nach Verfahren des Stands der Technik hergestellt werden, beispielsweise durch Pyrolyse der entsprechenden Fettsäure-Magnesiumsalze. Die Ketone können symmetrisch oder unsymmetrisch aufgebaut sein, vorzugsweise unterscheiden sich die beiden Reste R⁹ und R¹⁰ aber nur um ein Kohlenstoffatom und leiten sich von Fettsäuren mit 6 bis 22 Kohlenstoffatomen ab.

**Fettaldehyde.** Als Wachskörper geeignete Fettaldehyde entsprechen der Formel (VI),

R¹¹COH (VI)

in der R¹¹CO für einen linearen oder verzweigten Acylrest mit 24 bis 48, vorzugsweise 28 bis 32 Kohlenstoffatomen steht.

**Fettether.** Als Wachskörper kommen ferner Fettether der Formel (VII) in Frage,

R¹²-O-R¹³ (VII)

in der R¹² und R¹³ unabhängig voneinander für Alkyl- und/oder Alkenylreste mit 1 bis 22 Kohlenstoffatomen stehen, mit der Maßgabe, dass sie in Summe mindestens 24 und vorzugsweise 32 bis 48 Kohlenstoffatome aufweisen. Fettether der genannten Art werden üblicherweise durch saure Kondensation der entsprechenden Fettalkohole hergestellt. Fettalkohole mit 16 bis 22 Kohlenstoffatomen sind beispielsweise Cetylalkohol, Cetearylalkohol, Stearylalkohol, lsostearylalkohol, Oleylalkohol, Behenylalkohol und/oder Erucylalkohol.

**Fettcarbonate.** Als Wachskörper kommen weiterhin Fettcarbonate der Formel (VIII) in Betracht,

R¹⁴O-CO-OR¹⁵ (VIII)

in der R¹⁴ und R¹⁵ unabhängig voneinander für Alkyl-undloder. Alkenylreste mit 1 bis 22 Kohlenstoffatomen stehen, mit der Massgabe, dass sie in Summe mindestens 24 und vorzugsweise 32 bis 48 Kohlenstoffatome aufweisen. Die Stoffe werden erhalten, indem man beispielsweise Dimethyl- oder Diethylcarbonat mit den entsprechenden Fettalkoholen in an sich bekannter Weise umestert. Demzufolge können die Fettcarbonate symmetrisch oder unsymmetrisch aufgebaut sein. Vorzugsweise werden jedoch Carbonate eingesetzt, in denen R¹⁴ und R¹⁵ gleich sind und für Alkylreste mit 16 bis 22 Kohlenstoffatomen stehen. Beispiele sind Umesterungsprodukte von Dimethyl- bzw. Diethylcarbonat mit Cetylalkohol, Cetearylalkohol, Stearylalkohol, Isostearylalkohoi, Oleylalkohol, Behenylalkohol und/oder Erucylalkohol in Form ihrer Mono- und Diester bzw. deren technischen Mischungen.

**Epoxidringöffnungsprodukte.** Bei den Ringöffnungsprodukten handelt es sich um bekannte Stoffe, die üblicherweise durch säurekatalysierte Umsetzung von endständigen oder innenständigen Olefinepoxiden mit aliphatischen Alkoholen hergestellt werden. Die Reaktionsprodukte folgen vorzugsweise der Formel (IX), 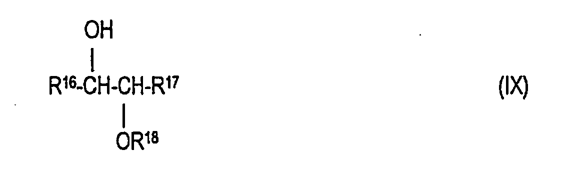 in der R¹⁶ und R¹⁷ für Wasserstoff oder einen Alkylrest mit 10 bis 20 Kohlenstoffatomen steht, mit der Maßgabe, dass die Summe der Kohlenstoffatome von R¹⁶ und R¹⁷ im Bereich von 10 bis 20 liegt und R¹⁸ für einen Alkyl- und/oder Alkenylrest mit 2 bis 22, vorzugsweise 12 bis 18 Kohlenstoffatomen und/oder den Rest eines Polyols mit 2 bis 15 Kohlenstoffatomen und 2 bis 10 Hdroxylgruppen steht. Typische Beispiele sind Ringöffnungsprodukte von α-Dodecenepoxid, α-Hexadecenepoxid, α-Octadecenepoxid, α-Eicosenepoxid, α-Docosenepoxid, i-Dodecenepoxid, i-Hexadecenepoxid, i-Octadecenepoxid, 1-Eicosenepoxid und/oder i-Docosenepoxid mit Ethanol, Laurylalkohol, Kokosfettalkohol, Myristylalkohol, Cetylafkohol, Cetearylalkohol, Stearylalkohol, Isostearylalkohol, Oleylalkohol, Elaidylalkohol, Petroselinylalkohol, Linolylalkohol, Linolenylalkohol, Behenylalkohol und/oder Erucylalkohol. Vorzugsweise werden Ringöffnungsprodukte von Hexa- und/oder Octadecenepoxiden mit Fettalkoholen mit 16 bis 18 Kohlenstoffatomen eingesetzt. Werden anstelle der Fettalkohole Polyole für die Ringöffnung eingesetzt, so handelt es sich beispielsweise um folgende Stoffe: Glycerin; Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton; technische Oligoglyceringemische mit einem Eigenkondensationsgrad von 1,5 bis 10 wie etwa technische Diglyceringemische mit einem Diglyceringehalt von 40 bis 50 Gew.-% ; Methylolverbindungen, wie insbesondere Trimethylolethan, Trimethylolpropan, Trimethylolbutan, Pentaerythrit und Dipentaerythrit; Niedrigalkylglucoside, insbesondere solche, mit 1 bis 8 Kohlenstoffen im Alkylrest wie beispielsweise Methyl- und Butylglucosid; Zuckeralkohole mit 5 bis 12 Kohlenstoffatomen wie beispielsweise Sorbit oder Mannit, Zucker mit 5 bis 12 Kohlenstoffatomen wie beispielsweise Glucose oder Saccharose; Aminozucker wie beispielsweise Glucamin.

**Fettsäuren.** Auch langkettige Fettsäuren eignen sich als Wachskörper und entsprechen vorzugsweise der Formel (X),

R¹⁹COOH (X)

in der R¹⁹CO für einen gegebenenfalls hydroxysubstituierten Acylrest mit 18 bis 54 Kohlenstoffatomen steht. Typische Beispiele sind Hydroxystearinsäure, Behensäure und/oder Calciumstearat bzw. Aluminiumstearat.

Es kann erfindungsgemäß ein Wachs oder ein Gemisch aus mehreren Wachsen verwendet werden.

Unter dem Begriff **"Öle"** (synonym verwendet: Ölkomponente) werden wasserunlösliche, bei 30°C flüssige, organische Verbindungen mit relativ niedrigem Dampfdruck bezeichnet. Das gemeinsame Merkmal der Öle ist nicht ihre übereinstimmende chemische Konstitution, sondern ihre ähnliche physikalische Konsistenz.

Als Ölkomponenten sind beispielsweise die nachstehend genannten Verbindungsklassen geeignet, soweit diese bei 30°C flüssig sind. So z.B. Guerbetalkohole auf Basis von Fettalkoholen mit 6 bis 18, vorzugsweise 8 bis 10 Kohlenstoffatomen (z. B. Eutanol® G), Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fetta!koho!en, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisosfearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, lsostearylisostearat, Isostearyloleat, Isostearylbehenat, Isostearyloleat, Oleylmyfistat, Oleylpalmitat, Oleylstearat, Oleylisostearat, Oleyloleat, Oleylbehenat, Oleyferucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat, Erucylerucat und Hexyldecylstearat (Eutanol® G 16 S). Daneben eignen sich Ester von linearen C₆-C₂₂-Fettsäuren mit verzweigten Alkoholen, insbesondere 2- Ethylhexanol, Ester von C₃-C₃₈-Alkylhydroxycarbonsäuren mit linearen oder verzweigten C₆-C₂₂-Feftalkoholen - insbesondere Dioctylmalat -, Ester von linearen und/oder verzweigten Fettsäuren mit mehrwertigen Alkoholen (wie z.B. Propylenglycol, Dimerdiot oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fetalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane wie z.B. 1 ,3-Dialkylcyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen (Hydagen® HSP, Sovermol® 750, Sovermol® 1102), Siliconöle (Cyclomethicone, Siliciummethicontypen u.a. und/oder aliphatische bzw. naphthenische Kohlenwasserstoffe, wie z.B. wie Mineralöl, Vaseline, Petrotatum, Squalan, Squalen oder Dialkylcyclohexane.

Als weitere Ölkörper kommen beispielsweise Silikonöle in Frage. Sie können als cyclische und/oder lineare Silikonöle vorliegen. Silikonöle sind hochmolekulare synthetische polymere Verbindungen, in denen Silicium- Atome über Sauerstoff-Atome ketten-und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel 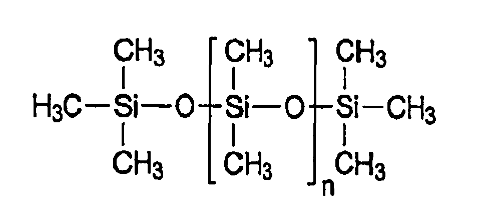 auszeichnen werden auch als Polydimethylsiloxan bzw. Dimethicon (INCI) bezeichnet. Dimethicone gibt es in verschiedenen Kettenlängen bzw. mit verschiedenen Molekulargewichten.

Vorteilhafte Polyorganosiloxane im Sinne der vorliegenden Erfindung sind beispielsweise Dimethylpolysiloxan [Poly(dimethylsiloxan)], welche beispielsweise unter den Handelsbezeichnungen Abil 10 bis 10 000 bei Evonik Goldschmidt erhältlich sind. Ferner vorteilhaft sind Phenylmethylpolysiloxan (INCI: Phenyl Dimethicon, Phenyl Trimethicon), cyclische Silikone (Octamethylcyclotetrasiloxan bzw. Decamethyloyclopentasiloxan), welche nach INCI auch als Cyclomethicon bezeichnet werden, aminomodifizierte Silikone (INCI: Amodimethicone) und Silikonwachse, z. B. Polysiloxan-Polyalkylen-Copolymere (INCI: Stearyl Dimethicon und Cetyl Dimethicon) und Dialkoxydimethylpolysiloxane (Stearoxy Dimethicon und Behenoxy Stearyl Dimethicon), welche als verschiedene Abil-Wax-'fypen bei Evonik Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan). Erfindungsgemäß besonders bevorzugte Silikone sind Dimethicon und Cyclomethicon.

Als Ölkomponenten eignen sich auch Polycarbonate, wie beispielsweise in WO 031041676 beschrieben, auf die hier ausdrücklich Bezug genommen wird.

Als Polycarbonate besonders geeignet ist das unter der INCI Bezeichnung Hydrogenated Dimer Dilinoleyl / Dimethylcarbonate Copolymer, weiches als Handelsprodukt Cosmedia® DC von Cognis GmbH erhältlich ist.

Die Dialkylcarbonate und Dialkylether können symmetrisch oder unsymmetrisch, verzweigt oder unverzweigt, gesättigt oder ungesättigt sein und lassen sich nach Reaktionen, die aus dem Stand der Technik hinlänglich bekannt sind, herstellen.

Erfindungsgemäß einsetzbar sind u.a. auch Kohlenwasserstoffe, vorzugsweise mit einer Kettenlänge 8 bis 40 C-Atomen. Sie können verzweigt oder unverzweigt sein, gesättigt oder ungesättigt. Unter diesen sind verzweigte, gesättigte C₈-C₄₀-Alkane bevorzugt. Es können sowohl Reinsubstanzen eingesetzt werden als auch Substanzgemische. Üblicherweise handelt es sich um Substanzgemische verschiedener isomerer Verbindungen. Zusammensetzungen, die Alkane mit 10 bis 30, vorzugsweise 12 bis 20, und besonders bevorzugt 16 bis 20 Kohlenstoffatome aufweisen, sind besonders geeignet, und unter diesen ein Gemisch aus Alkanen, welches wenigstens 10 Gew.-% verzweigte Alkane bezogen auf die Gesamtmenge der Alkane enthält. Vorzugsweise handelt es sich um verzweigte, gesättigte Alkane. Besonders gut geeignet sind Gemische aus Alkanen, welche mehr als 1 Gew.-% 5,8- Diethyldodecan und/oder mehr als 1 Gew.-% Didecen enthalten.

Es kann erfindungsgemäß ein Öl oder ein Gemisch aus mehreren Ölkomponenten verwendet werden.

### AntiperspirantlDesodorant-Wirkstoff

Erfindungsgemäß sind als Antiperspirant/Desodorant-Wirkstoff alle Wirkstoffe geeignet, die Körpergerüchen entgegen wirken, diese überdecken oder beseitigen. Körpergerüche entstehen durch die Einwirkung von Hautbakterien auf apokrinen Schweiß, wobei unangenehm riechende Abbauprodukte gebildet werden. Als Antiperspirant /Desodorant Wirkstoffe eignen sich insbesondere Verbindungen ausgewählt aus der Gruppe bestehend Antiperspirantien, Esteraseinhibitoren, bakterizide bzw. bakteriostatische Wirkstoffe und/oder schweißabsorbierende Substanzen.

Der AntiperspirantlDesodorant-Wirkstoff oder die Antiperspirant/Desodorant-Wirkstoffe ist/sind in üblicherweise verwendeten Mengen enthalten, üblicherweise in einem Anteil von 1 bis etwa 25 Gew.-%, bezogen auf das Gesamtgewicht der Antiperspirant/Desodorant-Zubereitung, bevorzugter 5 bis 22 Gew.-% oder 10 bis 20 Gew.-%. Es kann ein einziger Antiperspirant/Desodorant-Wirkstoff oder es können mehrere AntiperspirantlDesodorant-Wirkstoffe enthalten sein. Im letzteren Fall beziehen sich die obigen Prozentangaben auf die Gesamtmenge der enthaltenen Antiperspirant/Desodorant-Wirkstoffe.

### Antiperspirantien

Bei Antiperspirantien handelt es sich um Salze des Aluminiums, Zirkoniums oder des Zinks. Solche geeigneten antihydrotisch wirksamen Wirkstoffe sind z.B. Aiuminiumchiorid, Aluminiumchlorhydrat, Aluminiumdichlorhydrat, Aluminiumsesquichlorhydrat und deren Komplexverbindungen z. B, mit Propylenglycol-1,2. Aluminiumhydroxyallantoinat, Aiuminiumchioridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Vorzugsweise werden Aluminiumchlorhydrat, Aluminium-Zirkonium-tetrachlorohydrat, Aluminium-Zirkonium-pentachlor hydrat und deren Komplexverbindungen eingesetzt.

Die erfindungsgemäßen Zubereitungen können die Antiperspirantien bspw. in Mengen von 1 bis 25, vorzugsweise 5 bis 22 und insbesondere 8 bis 20 Gew.-% - bezogen auf das Gesamtgewicht der Antiperspirant-/Desodorant-Zubereitung - enthalten.

### Esteraseinhibitoren

Beim Vorhandensein von Schweiß im Achselbereich werden durch Bakterien extrazelluläre Enzyme - Esterasen, vorzugsweise Proteasen und/oder Lipasen - gebildet, die im Schweiß enthaltene Ester spalten und dadurch Geruchsstoffe freisetzen. Als Esteraseinhibitoren geeignet sind vorzugsweise Trialkylcitrate wie Trimethylcitrat, Tripropylcitrat, Trüsopropylcitrat, Tributylcitrat und insbesondere Triethylcitrat (Hydagen® CAT, Cognis GmbH, Düsseldod/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw. -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäure-diethylester, Hydroxycarbonsäuren und deren Ester wie beispielsweise Citronensäure, Äpfelsäure, Weinsäure oder Weinsäurediethylester sowie Zinkglycinat.

Die erfindungsgemäßen Zubereitungen können die Esteraseinhibitoren bspw. in Mengen von 0,01 bis 20, vorzugsweise 0,1 bis 10 und insbesondere 0,3 bis 5 Gew.-% - bezogen auf das Gesamtgewicht der Antiperspirant-/Desodorant-Zubereitung - enthalten.

### Bakterizide bzw. bakteriostatische Wirkstoffe

Typische Beispiele für geeignete bakterizide bzw. bakteriostatische Wirkstoffe sind insbesondere Chitosan und Phenoxyethanol. Besonders wirkungsvoll hat sich auch 5-Chlor-2-(2,4-dichlorphonoxy)-phenot erwiesen, das unter der Marke Irgasan® von der Ciba-Geigy, Basel/CH vertrieben wird. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)hamstoff, 2,4,4 '-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-Iod-2-propinylbufylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Famesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Sallcylsäure-n-decylamid.

Die erfindungsgemäßen Zubereitungen können die bakteriziden bzw. bakteriostatischen Wirkstoffe in Mengen von 0,01 bis 5 und vorzugsweise 0,1 bis 2 Gew.-% - bezogen auf das Gesamtgewicht der Antiperspirant-lDesodorant 2ubereitung - enthalten.

### Schweißabsorbierende Substanzen

Als schweißabsorbierende Substanzen kommen modifizierte Stärke, wie z.B. Dry Flo Plus (Fa. National Starch), Silikate, Talkum und andere Substanzen ähnlicher Modifikation, die zur Schweißabsorption geeignet erscheinen. Die erfindungsgemäßen Zubereitungen können die schweißabsorbierende Substanzen in Mengen von 0,1 bis 20, vorzugsweise 1 bis 20 und insbesondere 2 bis 8 Gew.-% - bezogen auf das Gesamtgewicht der Anüperspirant-/Desodorant-Zubereitung - enthalten.

### Emulgator

In einer Ausführungsform der Erfindung enthalten die erfindungsgemäßen Zubereitungen mindestens einen Emulgator.

Die erfindungsgemäßen Zubereitungen enthalten den/die Emulgator(en) üblicherweise in einer Menge von 0 bis 40 Gew.-%, vorzugsweise 0,1 bis 20 Gew.-% vorzugsweise 0,1 bis 15 Gew.-% und insbesondere 2 bis 10 Gew.-%, bezogen auf das Gesamtgewicht der Zubereitung.

Jedem Emulgator wird ein sogenannter HLB-Wert (eine dimensionslose Zahl zwischen 0 und 20) zugeschrieben, der angibt, ob eine bevorzugte Wasser- oder Öllöslichkeit vorliegt. Zahlen unter 9 kennzeichnen bevorzugt öllösliche, hydrophobe Emulgatoren, Zahlen über 11 wasserlösliche, hydrophile Emulgatoren. Der HLB-Wert sagt etwas über das Gleichgewicht der Grösse und Stärke der hydrophilen und der lipophilen Gruppen eines Emulgators aus. Der HLB-Wert eines Emulgators lässt sich auch aus Inkrementen errechnen, wobei die HLB-Inkremente für die verschiedenen hydrophilen und hydrophoben Gruppen, aus denen sich ein Molekül zusammensetzt. In der Regel kann er Tabellenwerken (z. B. H. P. Fiedler, Lexikon der Hilfsstoffe für Pharmazie, Kosmetik und angrenzende Gebiete, Editio Cantor Verlag, Aulendorf, 4. Aufl. 1996) oder den Herstellerangaben entnommen werden. Die Löslichkeit des Emulgators in den beiden Phasen bestimmt praktisch den Emulsionstyp. Ist der Emulgator besser in Wasser löslich erhält man eine O/W-Emulsion. Hat der Emulgator hingegen eine bessere Löslichkeit in der Ölphase entsteht unter sonst gleichen Herstellungsbedingungen eine W/O-Emulsion.

In einer Ausführungsform der Erfindung enthält die erfindungsgemäße Zubereitung mehr als einen Emulgator. Der Fachmann setzt in Abhängigkeit der übrigen Komponenten übliche Emulgator Systeme (wie z.B. Emulgator und Co-Emulgator) ein.

### Nicht-ionische Emulgatoren

Zur Gruppe der nicht-ionischen Emulgatoren gehören beispielsweise:
(1) Anlagerungsprodukte von 2 bis 50 Mol Ethylenoxid und/oder 1 bis 20 Mol Propylenoxid an lineare Fettalkohole mit 8 bis 40 C-Atomen, an Fettsäuren mit 12 bis 40 C-Atomen und an Alkylphenole mit 8 bis 15 Atomen in der Alkylgruppe.
(2) C₁₂-C₁₈-Feftsäuremono- und -diester von Anlagerungsprodukten von 1 bis 50 Mol Ethylenoxid an Glycerin.
(3) Sorbitanmono- und -diester von gesättigten und ungesättigten Fettsäuren mit 6 bis 22 Kohlenstoffatomen und deren Ethylenoxidanlagerungsprodukte.
(4) Alkylmono- und -oligoglycoside mit 8 bis 22 Kohlenstoffatomen im Alkylrest und deren ethoxylierte Analoga.
(5) Anlagerungsprodukte von 7 bis 60 Mol Ethylenoxid an Ricinusöl und/oder gehärtetes Ricinusöl.
(6) Polyol- und insbesondere Polyglycerinester, wie z. B. Polyolpoly-12-hydroxystearate, Poiygtycerinpoiyricinoieat, Polyglycerindüsostearat oder Polyglycerindimerat. Ebenfalls geeignet sind Gemische von Verbindungen aus mehreren dieser Substanzklassen.
(7) Anlagerungsprodukte von 2 bis 15 Mol Ethylenoxid an Ricinusöl und/oder gehärtetes Ricinusöl.
(8) Partialester auf Basis linearer, verzweigter, ungesättigter bzw. gesättigter C₆-C₂₂-Fettsäuren. Ricinolsäure sowie 12-Hydroxystearinsäure und Polyglycerin, Pentaerythrit, Dipentaerythrit, Zuckeralkohole (z. B. Sorbit), Alkylglucoside (z. B. Methylglucosid, Butylglucosid, Laurylglucosid) sowie Polyglucoside (z. B. Cellulose), oder Mischester wie z. B. Glycerylstearatcitrat und Glycerylstearatlactat.
(9) Polysiloxan-Polyalkyl-Polyether-Copolymere bzw. entsprechende Derivate.
(10) Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. Je nach Ethoxylierungsgrad handelt es sich um W/O- oder O/W-Emulgatoren. C_{12/18}-Fettsäuremono- und -diester von Anlagerungsprodukten von Ethylenoxid an Glycerin sind als Rückfettungsmittel für kosmetische Zubereitungen bekannt.

Erfindungsgemäß besonders gut geeignete und milde Emulgatoren sind Polyolpoly-12-hydroxystearate und Abmischungen davon, welche beispielsweise unter den Marken "Dehymuls^{®} PGPH" (W/O-Emulgator) oder "Eumulgin^{®}VL 75" (Abmischung mit Lauryl Glucosides im Gewichtsverhältnis 1:1, O/W-Emulgator) oder Dehymuls^{®} SBL (WIO-Emulgator) von der Cognis Deutschland GmbH vertrieben werden. In diesem Zusammenhang sei insbesondere auf das Europäische Patent EP 766 661 B1 verwiesen. Die Polyolkomponente dieser Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen.

Besonders bevorzugte Emulgatoren sind z. B. Cetyl Dimethicone Copolyol (z.B. Abil EM-90), Polyglyceryl-2 Dipolyhydroxystearate (z.B. Dehymuls PGPH), Polyglycerin-3-Dilsostearate (z.B. Lameform TGI), Polyglyceryl-4 Isostearate (z.B. Isolan GI 34), Polyglyceryl-3 Oleate (z.B. Isolan GO 33), Diisostearoyl Polyglyceryl-3 Diisostearate (z.B. Isolan PDI), Polyglyceryl-3 Methylglucose Distearate (z.B. Tego Care 450), Polyglyceryl-3 Beeswax (z.B. Cera Bellina), Polyglyceryl-4 Caprate (z.B. Polyglycerol Caprate T2010/90), Polyglyceryl-3 Cetyl Ether (z.B. Chimexane NL), Polyglyceryl-3 Distearate (z. B. Cremophor GS 32) und Polyglyceryl Polyricinoleate (z.B. Admul WOL 1403), Glyceryl Oleate (z.B. Monomuls 90-0 18), Alkyl Glucoside (z.B. Plantacare 1200, Emulgade PL 68150, Montanov 68, Tego Care CG 90, Tego Glucosid L 55), Methyl Glucose Isostearate (z.B. Tego Care IS), Methyl Glucose Sesquistearate (Tego Care PS), Sodium Cocoyl Hydrolyzed Wheat Protein (z.B. Gluadin WK), Potassium Cetyl Phosphate (z.B. Amphisol K, Crodafos CKP), Sodium Alkylsulfate (z.B. Lanette E), Sucrose Ester (z.B. Crodesta F-10, F-20, F-50, F-70, F-110, F-160, SL-40, Emulgade® Sucro), ethoxylierte und/oder propoxylierte Fettalkohole Fettsäuren, Rizinusöle bzw. hydrierte Rizinusöle (z.B. Eumulgin B2, B2, B3, L, HRE 40, HRE 60, RO 40, Cremophor HRE 40, HRE 60, L, WO 7, Dehymuls HRE 7, Arlacel 989), PEG-30 Dipolyhydroxystearate (z.B. Arlacel P 135, Dehymuls LE), Sorbitan Ester, Sorbitan Ester ethoxyliert und/oder propoxyliert sowie deren Gemische. Ein besonders effektives Gemisch besteht aus Polyglyceryl-2 Dipolyhydroxystearate und Lauryl Glucoside und Glycerin (z.B. Eumulgin VL 75). Geeignet sind weiterhin Polyglyceryl-4 Diisostearate/ Polyhydroxystearate/Sebacate (Isolan^{®} GPS), Diisostearoyl Polyglyceryl-3 Diisostearate (z. B. Isolan PDI), Alkalisalze Acylglutamate (z.B. Eumulgin SG).

Als lipophile W/O-Emulgatoren eignen sich prinzipiell Emulgatoren mit einem HLB-Wert von 1 bis 8, die in zahlreichen Tabellenwerken zusammengefaßt und dem Fachmann bekannt sind. Einige dieser Emulgatoren sind beispielsweise in Kirk-Othmer,"Encyclopedia of Chemical Technology", 3. Aufl., 1979, Band 8, Seite 913. aufgelistet. Für ethoxylierte Produkte lässt sich der HLB-Wert auch nach folgender Formel berechnen: HLB = (100 - L) : 5, wobei L der Gewichtsanteil der lipophilen Gruppen, d. h. der Fettalkyl- oder Fettacylgruppen in Gewichtsprozent, in den Ethylenoxidaddukten ist.

Besonders vorteilhaft aus der Gruppe der W/O-Emulgatoren sind Partialester von Polyolen, insbesondere von C₄-C₆-Polyolen, wie beispielsweise Partialester des Pentaerythrits oder Zuckerestem, z. B. Saccharosedistearat, Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoieat, Sorbitantriricinoleat, Sorbitanmonohydroxystearat, Sorbitansesquihydroxystearat, Sorbitan-dihydroxystearat, Sorbitantrihydroxystearat, Sorbitanmonotarkat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbilansesquimaleat, Sorbitandimaleat, Sorbitan-trimaleat sowie deren technische Gemische. Als Emulgatoren geeignet sind auch Anlagerungsprodukte von 1 bis 30, vorzugsweise 5 bis 10 Mol Ethylenoxid an die genannten Sorbitanester.

Je nach Formulierung kann es vorteilhaft sein, zusätzlich wenigstens einen Emulgator aus der Gruppe nicht-ionischer O/W-Emulgatoren (HLB-Wert: 8 - 18) und/oder Solubilisatoren einzusetzen. Hierbei handelt es sich beispielsweise um die bereits einleitend erwähnten Ethylenoxid-Addukte mit einem entsprechend hohen Ethoxylierungsgrad, z. B. 10 - 20 Ethylenoxid-Einheiten für O/W-Emulgatoren und 20 - 40 Ethylenoxid-Einheiten für sogenannte Solubilisatoren. Erfindungsgemäß besonders vorteilhaft als O/W-Emulgatoren sind Ceteareth-12 und PEG-20 Stearat. Als Solubilisatoren bevorzugt geeignet sind Eumulgin^{®} HRE 40 (INCI: PEG-40 Hydrogenated Castor Oil), Eumulgin^{®} HRE 60 (INCI: PEG-60 Hydrogenated Castor Oil), Eumulgin^{®} L (INCI: PPG-1-PEG-9 Laurylglycolether), sowie Eumulgin^{®} SML 20 (INCI: Polysorbat-20).

Nicht-ionische Emulgatoren aus der Gruppe der Alkyloligoglycoside sind besonders hautfreundlich und daher bevorzugt als O/W-Emulgatoren geeignet. C₈-C₂₂-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 22 C-Atomen. Bezüglich des Glycosidrestes gilt, dass sowohl Monoglycoside, bei denen ein zyklischer Zuckerrest glycosidisch an den Fettalkohol gebunden ist, als auch oligomere Glycoside mit einem Oigomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare^{®} zur Verfügung stehen, enthalten eine glucosidisch gebundene C₈-C₁₆-Alkylgruppe an einem Oligoglucosidrest, dessen mittlerer Oligomerisationsgrad bei 1 bis 2 liegt. Auch die vom Glucamin abgeleiteten Acylglucamide sind als nicht-ionische Emulgatoren geeignet. Erfindungsgemäß bevorzugt ist ein Produkt, das unter der Bezeichnung Emulgade^{®} PL 68/50 von der Cognis Deutschland GmbH vertrieben und ein 1:1-Gemisch aus Alkylpolyglucosiden und Fettalkoholen darstellt. Erfindungsgemäß vorteilhaft einsetzbar ist auch ein Gemisch aus Lauryl Glucoside, Polyglyceryl-2-Dipolyhydroxystearate, Glycerin und Wasser, das unter der Bezeichnung Eumulgin^{®} VL 75 im Handel ist.
Als Emulgatoren kommen weiterhin Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als Emulgatoren können beispielsweise Silikonemulgatoren enthalten sein. Diese können beispielsweise aus der Gruppe der Alkylmethiconcopolyole und/oder Alkyl-Dimethiconcopolyole gewählt werden, insbesondere aus der Gruppe der Verbindungen, welche gekennzeichnet sind durch die folgende chemische Struktur: 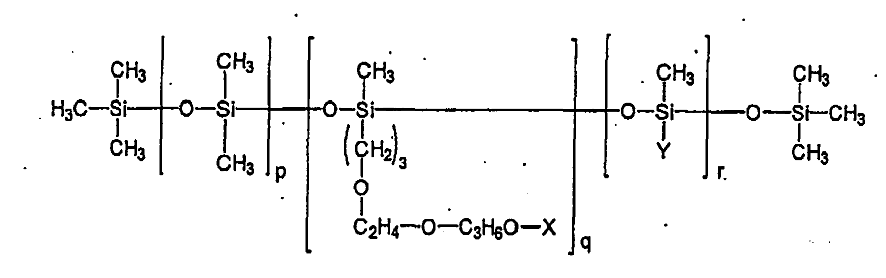 bei welcher X und Y unabhängig voneinander gewählt werden aus der Gruppe H (Wasserstoff) sowie der verzweigten und unverzweigten Alkylgruppen, Acylgruppen und Alkoxygruppen mit 1-24 Kohlenstoffatomen, p eine Zahl von 0-200 darstellt, q eine Zahl von 1-40 darstellt, und r eine Zahl von 1-100 darstellt.

Ein Beispiel für besonders vorteilhaft im Sinne der vorliegenden Erfindung zu verwendende Silikonemulgatoren sind Dimethiconcopolyole, welche von Evonik Goldschmidt unter den Warenbezeichnungen AXIL® B 8842, ABIL® B 8843, ABIL® B 8847, ABIL® B 8851, ABIL® B 8852, ABIL® B 8863, ABIL® B 8873 und ABIL®B 88183 verkauft werden. Ein weiteres Beispiel für besonders vorteilhaft im Sinne der vorliegenden Erfindung zu verwendende grenzflächenaktiven Substanzen ist das Cetyl PEG/PPG-1011 Dimethicone (Cetyl Dimethiconcopolyol), welches von EvonikGoldschmidt unter der Warenbezeichnung ABIL® EM 90 verkauft wird. Ein weiteres Beispiel für besonders vorteilhaft im Sinne der vorliegenden Erfindung zu verwendende grenzflächenaktiven Substanzen ist das Cyclomethicon Dimethiconcopolyol, welches von Evonik Goldschmidt unter der Warenbezeichnung ABIL®EM 97 und ABIL®WE 09 verkauft wird. Weiterhin hat sich als ganz besonders vorteilhaft der Emulgator Lauryl PEG/PPG-18/18 Methicone (Laurylmethiconcopolyol) herausgestellt, welcher unter der Warenbezeichnung Dow Corning® 5200 Formulation Aid von der Gesellschaft Dow Corning Ltd. erhältlich ist. Ein weiterer vorteilhafter Silikonemulgator ist Octyl Dimethicon Ethoxy Glucosid der Firma Wacker.

Erfindungsgemäß können die Zubereitungen alternativ oder zusätzlich anionische, amphotere und insbesondere kationische Emulgatoren enthalten.

Beispiele anionischer Emulgatoren sind Fettsäureslaze und Abmischungen von Fettsäuren und deren Salzen sowie Alkylsulfate (z.B. Natriumcetearylsulfat) und Alkylphosphate (z.B. Cetylphosphat).

Beispiele kationischer Emulgatoren sind quaternäre Ammoniumverbindungen.

### Polymere

In einer Ausführungsform der Erfindung enthalten die erfindungsgemäßen Zubereitungen mindestens ein Polymer.

Die erfindungsgemäßen Zubereitungen enthalten das/die Polymere(n) üblicherweise in einer Menge von 0 bis 20 Gew.-%, vorzugsweise 0,1 bis 15 Gew.-% und insbesondere 0,1 bis 10 Gew.-%, bezogen auf das Gesamtgewicht der Zubereitung.

Geeignete kationische Polymere sind beispielsweise kationische Cellulosederivate, wie z. B. eine quaternierte Hydroxyethylcellulose, die unter der Bezeichnung Polymer JR 400^{®} von Amerchol erhältlich ist, kationische Stärke, Copolymere von Diallylammoniumsalzen und Acrylamiden quaternierte UnylpyrroüdonNinylimidazol-Polymere, wie z.B. Luviquat^{®} (BASF), Kondensationsprodukte von Polyglycolen und Aminen, quaternierte Kollagenpolypeptide, wie beispielsweise Lauryldimonium hydroxy-propyl hydrolyzed collagen (Lamequat^{®}L/Grünau), quaternierte Weizenpolypepfide, Polyethylenimin, kationische Siliconpolymere, wie z. B. Amidomethicone, Copolymere der Adipinsäure und Dimefhylaminohydroxypropyldiethylentriamin (Cartaretine^{®}/ Sandoz), Copolymere der Acrylsäure mit Dimethyldiallylammoniumchlorid (Merquat^{®} 550/ Chemviron), Polyaminopolyamide, kationische Chitinderivate wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z. B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z. B. Mirapol^{®} A-15, Mirapol^{®} AD-1, Mirapol^{®} AZ-1 der Firma Miranol.

Als anionische, zwitterionische, amphotere und nichtionische Polymere kommen beispielsweise Vinylacetat/Crotonsäure-Copolymere. Vinylpyrroildon/Vinylacrylat-Copolymere, Vinylacetat/Butylmaleat/ lsobomylacrylat-Copolymere, Methylvinylether/Maleinsäureanhydrid-Copolymere und deren Ester, unvemetzte und mit Polyolen vernetzte Polyacrylsäuren, Acrylamidopropyltrimethylammoniumchlorid/ Acrylat-Copolymere, Octylacrylamid/Methylmethacrylat/tert. Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacryl/Vinylcaprolactam-Ter polymere sowie gegebenenfalls derivatisierte Celluloseether und Silicone in Frage.

Als Polymere eigen sich ebenso Polysaccharide, insbesondere Xanthan-Gum, Guar-Guar, Agar-Agar, Alginate und Tylosen sowie beispielsweise Aerosil-Typen (hydrophile Kieselsäuren), Carboxymethylcellulose und Hydroxyethyl- und Hydroxypropylcellulose, Polyvinylalkohol, Polyvinylpyrrolidon und Bentonite wie z. B. Bentone^{®} Gel VS-5PC (Rheox).

Ebenso geeignet sind sogenannte **quaternäre Polymere**, z.B. mit der INCI - Bezeichnung Polyquatemium-37, die der folgenden allgemeinen Formel entsprechen:

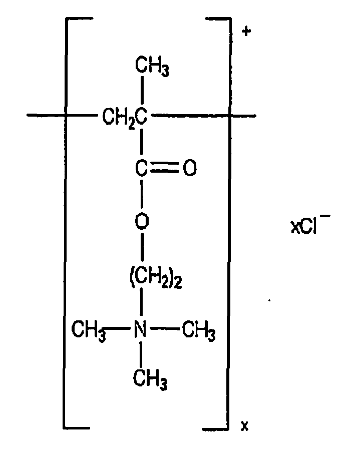

Alternativ können auch andere Dialkylaminoalkyl (meth)acrylate sowie deren durch Alkylierung oder Protonierung erhältliche Ammoniumsalze oder Dialkylaminoalkyl (meth)acrylamide sowie deren durch Alkylierung oder Protonierung erhältliche Ammoniumsalze eingesetzt werden. Besonders bevorzugt sind Polymere enthaltend MAPTAC, APTAC, MADAME, ADAME, DMAEMA und TMAEMAC. Darüberhinaus können auch Co-polymere mit anionischen, weiteren kationischen oder ungeladenen Monomeren erfindungsgemäß eingesetzt werden, insbesondere solche, die neben den genannten Alkylaminoalkyl (meth)acrylat oder -(meth)acrylamid Monomeren zusätzlich (Meth)acrylsäure und/oder 2-Acrylamido-2-methyl-propansulfonsäure und/oder Acrylamid und/oder Vinylpyrrolidon und/oder Alkyl(meth)acrylate enthalten. Beispielhaft seien solche Polymere mit der INCI Bezeichnung Polyquatemium-11, Polyquatemlum-13, Polyquatemium-14, Polyquatemium-15, Polyquatemium-28, Polyquatemlum-32, Polyquatemium-43, Polyquatemium-47 genannt.

Neben den genannten Bestandteilen können andere, in kosmetischen Zubereitungen und insbesondere Antiperspirant/Deodorant-Formulierungen übliche Bestandteile in üblichen Anteilen enthalten sein. Hierzu gehören z.B. Konservierungsmittel, biogene Wirkstoffe, Verdickungsmittel, Überfettungsmittel, Stabilisatoren, Polymere, Antioxidantien, Filmbildner, Quellmittel, Insektenrepellentien, Hydrotrope, Solubilisatoren, Parfümöle, Farbstoffe, UV-Filter, Pigmente etc.

Die erfindungsgemäßen Antiperspirant/Deodorant-Zubereitungen enthalten die weiteren Bestandteile üblicherweise in Mengen von insgesamt < 25 Gew.%, insbesondere < 20 Gew.%, bezogen auf das Gesamtgewicht der Antiperspirant-/Desodorant-Zubereitung.

Als Konservierungsmittel eignen sich beispielsweise Phenoxyethanol, Formaldehydlösung, Parabene, Mischungen aus Phenoxyethanol und Ethylhexylglycerin (wie sie beispielsweise unter dem Handelsnamen Euxyl PE 9010 erhältlich sind) oder Sorbinsäure sowie die unter der Bezeichnung Surfacine® bekannten Silberkomplexe und die in Anlage 6, Teil A und B der Kosmetikverordnung aufgeführten weiteren Stoffklassen.

In einer bevorzugten Ausführungsform der Erfindung wird das Konservierungsmittel ausgewählt aus der Gruppe bestehend aus Phenoxyethanol, Formaldehydlösung, Parabene, organische Säuren und Mischungen daraus, gegebenenfalls in Kombination mit Pentandiol und/oder Ethylhexylglycerin.

In einer Ausführungsform der Erfindung enthalten die erfindungsgemäßen Zubereitungen als weiteren Bestandteil mindestens einen biogenen Wirkstoff. Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

In einer bevorzugten Ausführungsform der Erfindung enthalten die erfindungsgemäßen Zubereitungen als biogenen Wirkstoff mindestens eine Verbindung ausgewählt aus Vitaminen, Allantoin, Bisabolol und Pflanzenextrakten.

In einer bevorzugten Ausführungsform der Erfindung enthalten die erfindungsgemäßen Zubereitungen als biogenen Wirkstoff mindestens eine Verbindung ausgewählt aus Tocopherol, Tocopherolacetat, Tocopherolpalmitat, Ascorbinsäure, (Desoxy)Ribonucleinsäuren und deren Fragmentierungsprodukten, β-Glucanen, Retinol, Bisabolol, Allantoin, Phytantriol, Panthenol, AHA-Säuren, Aminosäuren, Ceramiden, Pseudoceramiden, essentiellen Ölen, Pflanzenextrakten, wie z. B. Aloe vera, Prunusextrakt, Bambaranussextrakt und Vitaminkomplexe und Mischungen daraus.

In einer Ausführungsform der Erfindung enthalten die erfindungsgemäßen Antiperspirant/Deodorant-Zubereitungen als weiteren Bestandteil mindestens ein Verdickungsmittel.

Als Verdickungsmittel eignen sich beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylund Hydroxypropylcellulose, Polyvinylalkohol, Polyvinylpyrrolidon und Bentonite wie z. B. Bentone® Gel VS-5PC (Rheox).

Als Insekten-Repellentien kommen beispielsweise N,N-Diethyl-m-toluamid, 1 ,2-Pentandiol oder 3-(N-n-Butyl-N-acetyl-amino)-propionsäureethylester), welches unter der Bezeichnung Insect Repellent® 3535 von der Merck KGaA vertrieben wird, sowie Butylacetylaminopropionate in Frage.

Als Parfümöle seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage

Die weiteren Bestandteile der Zubereitungen (wie z.B. Konservierungsmittel, kosmetische Wirkstoffe, UV-Filter etc.) werden je nach ihrer Löslichkeit entweder über die Wasserphase oder über die lipophile Phase zugegeben.

Als Stabilisatoren können Metallsalze von Fettsäuren, wie z. B. Magnesium-, Aluminium- und/oder Zinkstearat bzw. -ricinoleat eingesetzt werden.

Zur Verbesserung des Fließverhaltens können ferner Hydrotrope, wie beispielsweise Ethanol, lsopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

### Wässriger Anteil:

Die erfindungsgemäßen Zubereitungen können üblicherweise 40 bis 80, bevorzugter 60 bis 80 Gew.-% wässrigen Anteil, bezogen auf das Gesamtgewicht der Zubereitung, enthalten.

Die erfindungsgemäßen Zubereitungen liegen im Allgemeinen als Emulsionen vor, in der Regel in Form von Gelen oder Gelcremes (nicht transparente Gele). Geeignete Formen der erfindungsgemäßen Zubereitungen sind z.B. Antitranspirant-/Deosprays, Antitranspirant/Deolotionen, Antitranspirant-/Deoroll-ons, Antitranspirant-/Deogele, Antitranspirant-/Deocremes oder Antitranspirant-/Deostifte.

### Beispiele

Die folgenden Beispiele sollen die vorliegende Erfindung näher erfäutern.

Es wurden die in Tabelle 1 gezeigten AP/Deo-Fonnutierungen nach üblichen Verfahren als Emulsionen hergestellt.

Die sensorischen Bewertungen (Trockenheit und Stumpfheit) erfolgten durch geschulte Testpersonen. Diese Bewertungen wurden jeweils 2 Minuten nach Auftragen der jeweiligen Zubereitung bewertet.

**Tab. 1: Zusammensetzung und Bewertung erfindungsgemäßer AP/Deo-Zubereitungen und von Vergleichsrezepturen**

| **Inhaltsstoffe** | **Schmelzpunkt der Wachse [°C]** | **Vergleichsbeispiel 1** | **Beispiel 1** | **Vergleichsbeispiel 2** | **Beispiel 2** |
|---|---|---|---|---|---|
| Steareth-2 | 55 | 3,00 | 3,00 | 3,00 | 3,00 |
| Ceteareth-12 | 36 | 1,25 | 1,25 | 1,25 | 1,25 |
| Ceteareth-20 | 40 | 0,75 | 0,75 | 0,75 | 0,75 |
| Cetylpalmitat | 46 | - | 1,0 | - | 2,25 |
| Myristylmyristat | 38 | - | 1,0 | - | 2,25 |
| Distearylether | 60 | - | 1,0 | - | - |
| Dicaprylcarbonat | | 3,0 | - | 4,50 | - |
| Dimethicone 200 (100cs) | | 1,0 | 1,0 | 1,50 | 1,50 |
| | | | | | |
| Aluminiumchiorhydrat | | 20,0 | 20,0 | 20,0 | 20,0 |
| Wasser, Konservierung | | 71,0 | 71,0 | 69,0 | 69,0 |
| | | | | | |
| Wachsanteil, bezogen auf lipophile Phase [Gew.-%] | | 0 | 75,0 | 0 | 75,0 |
| | | | | | |
| Viskosität [Brookfield, RVF, Spindel 5,10 UpM; mPas] | | 4000 | 800 | 2700 | 800 |
| Bewertung der Trockenheit unter der Achsel: 2 Minuten nach Anwendung | | 1 | 4 | 2 | 4 |
| 1- sehr feucht | | | | | |
| 2 - feucht | | | | | |
| 3 - trocken | | | | | |
| 4 - sehr trocken | | | | | |
| Bewertung der Stumpfheit unter der Achsel: 2 Minuten nach Anwendung | | 4 | 4 | 4 | 4 |
| 1 - sehr stumpf | | | | | |
| 2 - stumpf | | | | | |
| 3 - glatt | | | | | |
| 4 - sehr glatt | | | | | |

Bei Dicaprylcarbonat und Dimethicone 200 handelt es sich um bei Raumtemperatur flüssige Ölkomponenten. Cetylpalmitat, Myristylmyristat und Distearylether sind erfindungsgemäß den Wachsen zuzuordnen.

Als Vergleichsbeispiele wurden Antiperspirant-/Desodorant-Zubereitungen gewählt, welche keine Wachskomponente in der lipophilen Phase aufweisen. In Vergleichsbeispiel 1 sind im Vergleich zu Beispiel 1 die festen Wachse Cetylpalmitat, Myristylpalmitat und Distearylether durch das bei Raumtemperatur flüssige Dicaprylcarbonat ersetzt. In Vergleichsbeispiel 2 sind im Vergleich zu Beispiel 2 die festen Wachse Cetylpalmitat und Myristylpalmitat durch das bei Raumtemperatur flüssige Dicaprylcarbonat ersetzt. Es zeigte sich klar, dass bei den erfindungsgemäßen Beispielen im Gegensatz zu den Vergleichsbeispielen schnell ein trockenes und gleichzeitig glattes Gefühl vorlag. Die Antiperspirant/Desodorant-Wirkung war in allen Beispielen und Vergleichsbeispielen gegeben.

## Patentansprüche

1. Antiperspirant/Desodorant-Zubereitungen, enthaltend:
a. einen Andperspirant-/Desodorant-Wirkstoff, und
b. eine lipophile Phase, die zu 50 Gew.-% oder mehr, bezogen auf das Gewicht der lipophilen Phase, aus Wachsen mit einem Schmelzpunkt von > 30°C gebildet ist.

2. Zubereitungen nach Anspruch 1, **dadurch gekennzeichnet, dass** die lipophile Phase zu etwa 70 bis etwa 80 Gew.-% aus Wachsen mit einem Schmelzpunkt > 30°C gebildet ist.

3. Zubereitungen nach einem der vorhergehenden Ansprüche, **dadurch gekennzeichnet, dass** der Anteil der lipophilen Phase, bezogen auf die Antiperspirant/Desodorant-Zubereitung, 2 - 15 Gew.-% beträgt.

4. Zubereitungen nach einem der vorhergehenden Ansprüche, **dadurch gekennzeichnet, dass** der Anteil des Antiperspirant-/Desodorant-Wirkstoff, bezogen auf die Antiperspirant/Desodorant- ' Zubereitung, 10-25 Gew.-% beträgt.

5. Zubereitungen nach einem der vorhergehenden Ansprüche, **dadurch gekennzeichnet, dass** es sich um Emulsionen handelt.

6. Zubereitungen nach einem der vorhergehenden Ansprüche in Form von Antitranspirant-/Deosprays, Antitranspirant-/Deoiotionen, Antitranspirant-/Deoroll-ons, Antitranspirant-/Deogelen, Antitranspirant-/Deocremes oder Antitranspirant-/Deostiften.

7. Verfahren zur Verbesserung der sensorischen Eigenschaften einer Antiperspirant/Desodorant-Zubereitung, **dadurch gekennzeichnet, dass** man die lipophile Phase derart einstellt, dass diese 50 Gew.-% oder mehr, bezogen auf das Gewicht der lipophilen Phase, Wachs(e) mit einem Schmelzpunkt von > 30°C enthält.
